(19) Europäisches Patentamt

European Patent Office

Office européen des brevets

(11) **EP 2 911 804 B1**

(12) **EUROPEAN PATENT SPECIFICATION**

(45) Date of publication and mention
of the grant of the patent:
**08.07.2020 Bulletin 2020/28**

(21) Application number: **13792526.9**

(22) Date of filing: **25.10.2013**

(51) Int Cl.:
***B05B 13/02*** *(2006.01)*     ***B05D 1/00*** *(2006.01)*
***B05D 1/26*** *(2006.01)*     ***A61M 25/10*** *(2013.01)*
***B05C 13/02*** *(2006.01)*     ***B05C 11/02*** *(2006.01)*
***B05C 5/02*** *(2006.01)*

(86) International application number:
**PCT/US2013/066810**

(87) International publication number:
**WO 2014/066760 (01.05.2014 Gazette 2014/18)**

(54) **APPARATUS AND METHODS FOR COATING MEDICAL DEVICES**

VORRICHTUNG UND VERFAHREN ZUR BESCHICHTUNG MEDIZINISCHER VORRICHTUNGEN

APPAREIL ET PROCÉDÉS POUR REVÊTIR DES DISPOSITIFS MÉDICAUX

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **25.10.2012 US 201261718358 P
07.12.2012 US 201261734788 P**

(43) Date of publication of application:
**02.09.2015 Bulletin 2015/36**

(73) Proprietor: **SurModics, Inc.
Eden Prairie, MN 55344-3523 (US)**

(72) Inventors:
• **CHAPPA, Ralph A.
Ham Lake, Minnesota 55304 (US)**
• **CARLSON, Mark F.
St. Louis Park, Minnesota 55426 (US)**
• **KLOKE, Timothy M.
Victoria, Minnesota 55386 (US)**

(74) Representative: **Potter Clarkson
The Belgrave Centre
Talbot Street
Nottingham NG1 5GG (GB)**

(56) References cited:
| | |
|---|---|
| WO-A2-2014/182833 | US-A- 4 743 252 |
| US-A1- 2005 233 061 | US-A1- 2009 018 643 |
| US-A1- 2009 317 537 | US-A1- 2010 055 294 |
| US-A1- 2010 070 020 | US-A1- 2011 238 011 |
| US-A1- 2011 253 170 | US-A1- 2011 311 713 |
| US-A1- 2012 258 246 | |

## Description

### Field of the Invention

The present invention relates to apparatus and methods for coating medical devices.

### Background of the Invention

Functional improvements to implantable or insertable medical devices can be achieved by coating the surface of the device. For example, a coating formed on the surface of the device can provide improved lubricity, improved biocompatibility, or drug delivery properties to the surface. In turn, this can improve movement of the device in the body, extend the functional life of the device, or treat a medical condition near the site of implantation. However, various challenges exist for the design and use of coating apparatus designed to provide coatings to medical devices.

Traditional coating methods, such as dip coating, are often undesirable as they may result in flawed coatings that could compromise the function of the device or present problems during use. These methods can also result in coating inaccuracies, which can be manifested in variable amounts of the coated material being deposited on the surface of the device. When a drug is included in the coating material, it is often necessary to deliver precise amounts of the agent to the surface of the device to ensure that a subject receiving the coated device receives a proper dose of the agent. It has been difficult to achieve a great degree of accuracy using traditional coating methods and machines.

One type of insertable medical device is a balloon catheter. Balloon catheter constructions are well known in the art and are described in various documents, for example, U.S. Pat. Nos. 4,195,637, 5,041,089, 5,087,246, 5,318,587, 5,382,234, 5,571,089, 5,776,101, 5,807,331, 5,882,336, 6,394,995, 6,517,515, 6,623,504, 6,896,842, and 7,163,523. Balloon catheters generally include four portions, the balloon, catheter shaft, guide wire, and manifold. A balloon catheter generally includes an elongated catheter shaft with an inflatable balloon attached to a distal section of the catheter shaft. At a proximal end of the catheter shaft, there is typically a manifold. At the manifold end, placement of the catheter can be facilitated using a guide wire. Guide wires are small and maneuverable when inserted into an artery. Once the guide wire is moved to the target location, the catheter with balloon portion is then fed over the guide wire until the balloon reaches the target location in the vessel. The balloon is typically inserted into the arterial lumen of a patient and advanced through the lumen in an unexpanded state. The balloon is then inflated when the catheter reaches target site resulting in application of mechanical force sufficient to cause vessel dilation. The balloon is typically inflated using a fluid, which is injected through an inflation port. The manifold can control the fluid introduction within shaft for expansion of the balloon. The mechanics of fluid transfer and introduction within balloons vary according to the specific design of the catheter, and are well known in the art.

US 2011/0311713 discloses a method for applying a coating to an implantable device. The method includes positioning an implantable device relative to an ultrasonic material delivery apparatus. The implantable device is rotated at a relative speed. The relative speed may be more than 120 revolutions per minute. US 2010/0070020 discloses an implantable medical device comprising a fibrous polymer body comprising a plurality of electrospun poly(urethane) fibers, a support filament wrapped around the body, an outer layer around the filament for adhering the filament to the body, the outer layer comprising a plurality of electrospun poly(urethane) fibers, and a polymer primer coating at least the fibers of the body. US 2010/0055294 discloses a method and apparatus for coating a medical device. The method can include (1) preparing a coating solution comprising a solvent, a therapeutic agent, and an additive; (2) loading a metering dispenser with the coating solution; (3) rotating the medical device about the longitudinal axis of the device and/or moving the medical device along the longitudinal or transverse axis of the device; (4) dispensing the coating solution from the metering dispenser onto a surface of the medical device and flowing the coating solution on the surface of the medical device while the medical device is rotating and/or linearly moving; and (5) evaporating the solvent, forming a substantially uniform coating layer on the medical device. US 2009/0018643 discloses a composition comprising a stent and a polymer scaffold. US 4743252 discloses composite grafts that have a porous wall structure to permit ingrowth thereinto under in vivo conditions but which include a generally non-porous membrane in the wall to prevent substantial fluid passage therethrough so as to provide an implantable porous graft that does not require preclotting prior to implantation. US 2011/0238011 describes coating processes for the manufacture of balloon catheters, in which a catheter balloon is mounted horizontally during a coating stage and is rotated about its longitudinal axis while a micro-dosing unit moves to and fro along the longitudinal axis of the catheter balloon. A syringe, a cannula, a tube or some other device can be used as the micro-dosing unit. US 2005/0233061 describes coating surfaces of medical devices using a coating head comprising at least one outlet orifice from which flows a coating material, to deposit at least one layer of coating material dispelled through the outlet orifice onto the surface of the medical device.

### Summary of the Invention

The invention provides a coating apparatus according to claim 1 and a method of coating according to claim 7. More generally, the present disclosure describes a coating apparatus, which is not part of the claimed invention, including a coating application unit including a fluid applicator; a first rotation mechanism and a second rotation mechanism; and a controller, wherein the controller causes the first rotation mechanism and the second rotation mechanism to rotate a medical device at substantially the same speed, wherein the speed is greater than 500 rotations per minute.

Also described, but not part of the invention, is a method of coating a medical device including rotating a medical device with a rotation mechanism at a speed of greater than 500 rotations per minute; contacting the medical device with a fluid applicator; and applying a coating solution to the device with the fluid applicator.

Also described is a medical device, which is not part of the claimed invention, including a shaft defining a lumen; and an inflatable balloon attached to the shaft; and a coating layer disposed over at least a portion of the shaft; wherein the surface of the shaft comprises high points and low points, wherein the thickness of the coating is substantially the same over both the high points and the low points.

Also described is a medical device, which is not part of the claimed invention, including a shaft defining a lumen; and an inflatable balloon attached to the shaft, the balloon comprising a plurality of longitudinal pleats; and a coating layer disposed over a portion of the balloon, wherein the coating layer defines apertures that correspond to the longitudinal pleats.

Also described is a coating apparatus, which is not part of the claimed invention, and which can include a motor, a rotating contact member, a fluid applicator, a fluid pump, and a base member. The fluid applicator can include an orifice. The orifice of the fluid applicator can be disposed adjacent to the rotating contact member. The rotating contact member can be in mechanical communication with the motor. The rotating contact member can be configured to rotate around a device to be coated that does not rotate. The rotating contact member can be configured to move along the lengthwise axis of a device to be coated. The fluid pump can be in fluid communication with the fluid applicator. The base member can support the rotating contact member and the fluid applicator.

Also described, but not part of the invention, is a method of coating a medical device which can include rotating a contact member around the outer diameter of a non-rotating medical device. The method can further include applying a coating solution to the outer diameter of the non-rotating medical device at a position adjacent to the contact member. The method can further include moving at least one of the contact member and the non-rotating medical device with respect to one another so that the contact member moves with respect to the lengthwise axis of the non-rotating medical device.

This summary is an overview of some of the teachings of the present application and is not intended to be an exclusive or exhaustive treatment of the present subject matter. Further details are found in the detailed description and appended claims. Other aspects will be apparent to persons skilled in the art upon reading and understanding the following detailed description and viewing the drawings that form a part thereof, each of which is not to be taken in a limiting sense. The scope of the present invention is defined by the appended claims.

### Brief Description of the Figures

The invention may be more completely understood in connection with the following drawings, in which:

FIG. 1 is a partial cross-sectional view of a device including a shaft with a central lumen.
FIG. 2A is a partial cross-sectional view of the substrate of a medical device.
FIG. 2B is a partial cross-sectional view of a medical device.
FIG. 3 is a partial cross-sectional view of a device including a shaft with a central lumen.
FIG. 4 is a schematic view of a portion of a medical device.
FIG. 5A is a partial cross-sectional view of the substrate of a medical device.
FIG. 5B is a cross-sectional view of a medical device.
FIG. 6 is a schematic perspective view of a coating apparatus.
FIG. 7 is a schematic perspective view of a coating apparatus.
FIG. 8 is a front elevational view of a fluid applicator.
FIG. 9 is a side elevational view of the fluid applicator of FIG. 8.
FIG. 10 is a top plan view of the fluid applicator of FIG. 8.
FIG. 11 is a schematic view of a fluid applicator.
FIG. 12 is a partial view of a medical device with a coating.
FIG. 13 is a partial view of a medical device with a coating.
FIG. 14 is a partial view of a medical device with a coating.
FIG. 15 is a partial view of a medical device with a coating.
FIG. 16 is a schematic view of a coating apparatus.
FIG. 17A is a front view of a drive shaft and rotating contact member of a coating apparatus.
FIG. 17B is a front view of a drive shaft and rotating contact member of a coating apparatus.
FIG. 18A is a schematic view of a coating apparatus.
FIG. 18B is a schematic view of a coating apparatus.
FIG. 19 is a schematic view of a coating apparatus.
FIG. 20 is a schematic view of a coating apparatus.
FIG. 21 is a schematic cross-sectional view of a rotating contact member.
FIG. 22 is a schematic cross-sectional view of a rotating contact member.
FIG. 23 is a schematic cross-sectional view of a rotating contact member.

FIG. 24 is a schematic view of a coating apparatus.

FIG. 25 is a schematic view of a coating apparatus.

FIG. 26 is a schematic view of a coating apparatus.

While the invention is susceptible to various modifications and alternative forms, specifics thereof have been shown by way of example and drawings, and will be described in detail. It should be understood, however, that the invention is defined by reference to the accompanying claims.

**Detailed Description of the Invention**

The embodiments of the present invention described herein are not intended to be exhaustive or to limit the invention to the precise forms disclosed in the following detailed description. Rather, the embodiments are chosen and described so that others skilled in the art can appreciate and understand the principles and practices of the present invention.

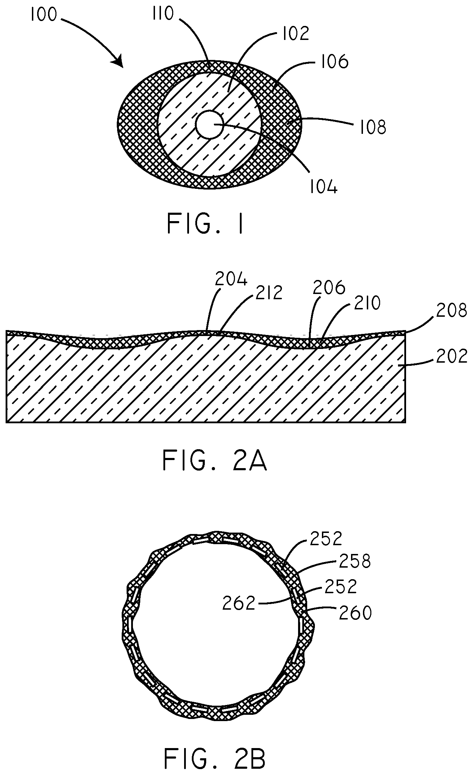

Standard coating techniques can include dip coating and/or spray coating. Applicants have observed, however, that standard techniques such as dip coating can result in various coating irregularities. By way of example, dip coating can result in coatings that are characterized by a thicker side and a thinner side. Referring to FIG. 1, a cross-sectional view is shown of a device 100 including a shaft 102 with a central lumen 104. The device 100 also includes a coating 106. However, the coating 106 is not disposed evenly around the circumference of the shaft 102. Rather, the coating 106 has a thick side 108 and a thin side 110. This can pose various problems including sub-optimal durability of the coating.

In addition, standard coating techniques can result coating irregularities, particularly where the surface of the object to be coated has some degree of surface topology variation. For example for objects with a surface having a variable topology (high points and low points), there is usually a larger amount of coating material that ends up over the low points and a lesser amount that ends up over the high points. Referring now to FIG. 2A, a partial cross-sectional view is shown of the substrate 202 of a medical device that includes high points 204 and low points 206. A coating 208 is disposed on the substrate 202. The coating includes thicker portions 210 and thinner portions 212. This type of coating pattern can be problematic because the higher points are also subject to a greater degree of friction during use than the low points and so a coating with these types of irregularities can be substantially less durable and more prone to release of particulate matter as the coating breaks down.

In the context of medical devices with gaps in between features, standard coating techniques can result in coatings that span the gaps (such as a webbing) and/or flow through to the inner diameter resulting in a coating configuration that is undesirable for some types of applications. Referring now to FIG. 2B, a partial cross-sectional view of a medical device is shown. The medical device includes a plurality of segments 252 separated from one another by gaps 260 (such as slots on the surface of a tube) and surrounding a central lumen. A coating material 258 is disposed on the device. However, the coating material is disposed within the gaps 260 and on the inner diameter surface 262 of the medical device. The coating material between the segments can be susceptible to breaking off and can therefore serve as a source of particulates. In addition, the presence of the coating on the inner diameter can be undesirable if different functional properties are desired between the inner and outer diameter of the medical device.

Coating apparatus as described herein can be used to apply coatings, including coatings with or without active agents, onto medical devices, such as onto the shafts and/or balloons of balloon catheters while addressing various shortcomings of standard coating techniques. Notably, coating apparatus as described herein can be used to coat medical devices having uniform coatings that are substantially even around the circumference of the medical device. This has been found to be possible even in the context of coating solutions that are extremely difficult to use in forming even coatings such coating solutions with high viscosities and coatings solutions with low viscosities.

In addition, coating apparatus as described herein can be used to coat medical devices having variable surface topology that result in coatings that are substantially more uniform in terms of the amount of coating applied when comparing high points and low points on the surface. In some arrangements, coating apparatus as described herein can be used to apply coatings wherein the typical pattern of more coating material over low points is actually reversed such that there is more coating material over high points.

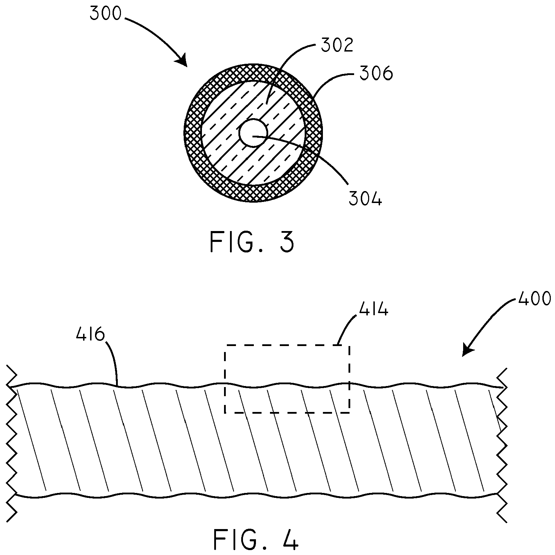

Coating apparatus as described herein may include rotation mechanisms that spin the medical device to be coated at a relatively high rate. Spinning of the medical device at a high rate results in a substantial centrifugal force being generated that acts to make the thickness of the coating substantially uniform when evaluating the coating thickness around the circumference of a medical device. Referring now to FIG. 3, a cross-sectional view is shown of a device 300 including a shaft 302 with a central lumen 304. The device 300 also includes a coating 306. However, in contrast to the coating 106 shown in FIG. 1, the coating 306 is disposed substantially evenly around the circumference of the shaft 302.

In addition, the substantial centrifugal force counteracts the tendency of coating material to pool in low points which is otherwise true in the context of lower speed spinning or stationary applications. Surprisingly, Applicants have discovered that high RPM spinning can be utilized without the coating solution being thrown off the surface of the medical device after it is applied. In other words, Applicants have discovered that very high rotations per minute can be used in the coating process without the centrifugal force getting so high that the coating material is cast off of the surface of the device being coated.

In some arrangements, the device to be coated is rotated at a speed of between 500 and 2000 rotations per minute (RPM). In some arrangements, the device to be coated can be rotated at a speed of between 600 and 1500 rotations per minute. In some arrangements, the device to be coated can be rotated at a speed of between 700 and 1000 rotations per minute. In some arrangements, the device to be coated can be rotated at a speed greater than 500, 600, 700, 800, 900, 1000, 1100, or 1200 rotations per minute.

Centrifugal force can be calculated according to the following equation (I):

$$Fc = 0.01097 \times M \times r \times n^2 \quad \text{(Equation I)}$$

where

Fc = centrifugal force (N (kg*m/s$^2$))
M = mass (kg)
r = radius (m)
n = RPM

As such, it can be seen that the force varies linearly with radius but non-linearly with RPM. For this reason, it has come as a surprising result that at such high RPM speeds that the coating is able to stay on the surface of the medical device being coated.

The radius of medical devices coated as described herein can vary. In some arrangements, the radius of the device coated is less than 5 cm, 4 cm, 3 cm, 2 cm, or 1 cm. In some arrangements, the radius of the device coated is between about 0.1 mm and 2 cm. In some arrangements, the radius of the device coated is between about 0.2 mm and 1 cm.

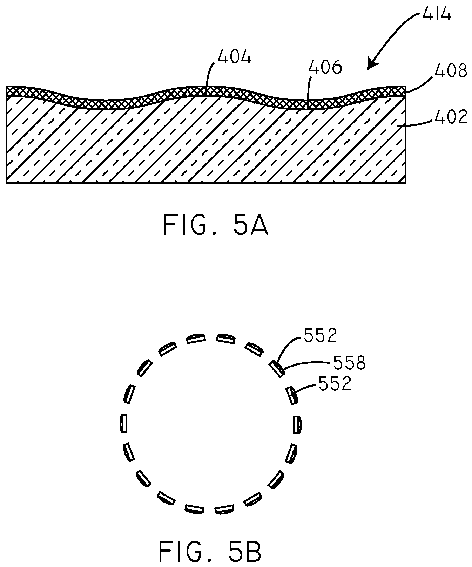

Referring now to FIG. 4, a schematic view is shown of a portion of a medical device 400. The surface 416 of the device 400 has relative high points and low points. Referring now to FIG. 5A, a partial cross-sectional view of a specific segment 414 of the device 400 is shown. The device 400 includes a coating 408 disposed on a substrate 402. The substrate surface includes high points 404 and low points 406. FIG. 5A is distinguished from that shown in FIG. 2 in that the coating 408 is substantially even above both high points 404 and low points 406. While the specific thickness of the coating can vary depending on the end use, it will be appreciated that the thickness can be from about 0.1 microns to about 50 microns or the thickness can be from 1 to 10 microns.

Exemplary medical devices represented by device 400 can include braided catheters. Braided catheters are typically used in applications that require high torque, burst pressure resistance pushability, steerability and kink resistance. Exemplary uses include EP mapping and ablation catheters, temperature sensing, pressure monitoring, robotic & optical catheters (available Creganna-Tactx Medical, Galway, Ireland).

Arrangements as described herein can be used to apply coatings to medical devices with gaps in between features such that the coating does not span the gaps and does not migrate to cover the inner diameter of the medical device. Referring now to FIG. 5B, a partial cross-sectional view of a medical device is shown. The medical device includes a plurality of segments 552 separated from one another by gaps and surrounding a central lumen. A coating material 858 is disposed on the device. However, in contrast to the medical device shown in FIG. 2B, the coating material is not disposed within the gaps and is not on the inner diameter surface 262 of the medical device.

In addition, arrangements as described herein can be used to apply coatings onto medical devices having porous substrates and/or surfaces and prevent and/or reduce the amount of coating migration from the surface into the porous material. In specific, the centrifugal force provided by the high RPM speeds can serve to at least partially counteract the forces (such as capillary action) that otherwise serve to cause coating materials to migrate into the porous material.

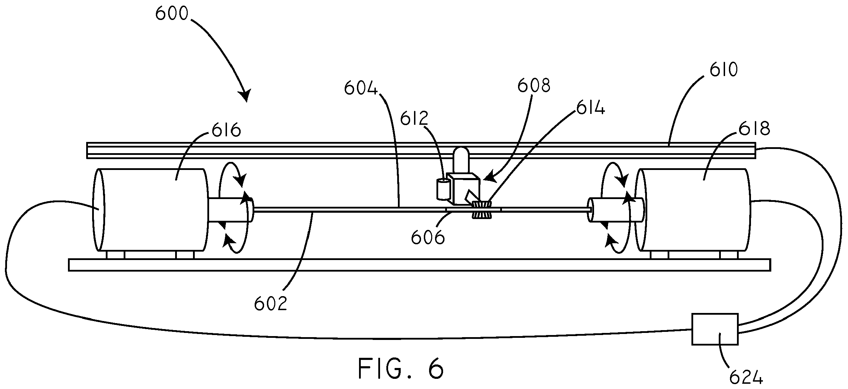

Referring now to FIG. 6, a schematic side view is shown of a coating apparatus 600. The coating apparatus 600 is shown in conjunction with a balloon catheter 602. The balloon catheter 602 can include a catheter shaft 604 and a balloon 606. The balloon 606 can assume a deflated configuration and an inflated configuration. The balloon catheter 602 can include a distal end and a proximal end. The balloon catheter 602 can include a proximal end manifold (not shown). The coating apparatus 600 includes a coating application unit 608. The coating application unit 608 can move along a support rail 610. However, it will be appreciated that the coating application unit 608 can remain stationary, and other components of the coating apparatus 600 can move.

Coating of the balloon catheter 602 can proceed starting at the proximal end of the and proceeding to the distal end. However, in other arrangements, coating of the balloon catheter 602 can occur starting at the distal end and proceeding to the proximal end. In some arrangements, coating can take place with a single pass of the coating application unit 608 with respect to the balloon catheter 602. However, in other arrangements, multiple passes of the coating application unit 608 with respect to the balloon catheter 602 can be made.

The coating application unit 608 can further include a fluid pump 612. The fluid pump 612 can be, for example, a syringe pump. The fluid pump 612 can be in fluid communication with components of the coating application unit 608 (such as the fluid applicator). The fluid pump 612 can operate to pump a coating solution at a rate sufficient to apply about 0.5 μl to about 10 μl of the coating solution per centimeter of length of the shaft or other portion of the device to be coated. The coating application unit 608 can further include a fluid applicator 614.

According to the invention, the fluid applicator is a contact fluid applicator (e.g., the fluid applicator makes physical contact with the medical device being coated). While not intending to be bound by theory, it is believed that contact fluid applicators can offer advantages in terms of precise control over the starting and stopping points of segments of coating applied to medical devices, reduced waste (such as material that may otherwise be lost as overspray), and/or control over total amounts of materials deposited onto medical devices (such as active agents and the like). Also described herein is a fluid applicator which is a non-contact fluid applicator; however this solution is not part of the invention. Details of exemplary fluid applicators are provided below.

In contrast to dip coating approaches, arrangements described herein can be highly efficient in terms of the use of coating reagents because there is no dead volume or residual volume associated with a container into which a device is dipped. Rather, the amounts of coating reagents used can closely track the amount actually deposited onto medical devices. In some arrangements, greater than 80% of the coating solutions consumed in the process of coating are deposited onto the medical devices being coated. In some arrangements, greater than 90% of the coating solutions consumed in the process of coating are deposited onto the medical devices being coated. In some arrangements, greater than 95% of the coating solutions consumed in the process of coating are deposited onto the medical devices being coated. In some arrangements, greater than 98% of the coating solutions consumed in the process of coating are deposited onto the medical devices being coated. In some arrangements, greater than 99% of the coating solutions consumed in the process of coating are deposited onto the medical devices being coated.

In some arrangements, the rate at which a coating solution is applied to the surface of the medical device can be dynamically changed based on the relative size of the portion being coated. By way of example, it will be appreciated that in order to provide a coating a given thickness, the relative amount of coating solution needed will change based on the diameter of the device to be coated with larger diameters requiring greater amounts of coating solution. As such, in various arrangements, the rate at which coating solution is applied to the surface of a medical device can be dynamically changed according to the diameter (or other size measure) of the portion currently being coated.

In some arrangements, a contact fluid applicator can be used to sense the size of the medical device being coated and this information can be used by a controller to dynamically calculate and appropriate rate of application of coating solution (or pumping rate).

The coating apparatus 600 further includes a first rotation mechanism 616 (and optionally a rotating balloon catheter fixture) and a second rotation mechanism 618 (and optionally a rotating balloon catheter fixture). The rotation mechanisms 616, 618 can be directly or indirectly coupled to the balloon catheter in order to rotate the balloon catheter 602 around its lengthwise (major) axis (about the central lumen of the catheter). The rotation mechanisms can spin at a high rate in order to generate a substantial centrifugal force pulling coating material in an outward direction away from the central lumen of the catheter. According to the invention, the medical device is rotated at a speed greater than 500 rotations per minute. In some arrangements, the balloon catheter can be rotated at a speed of between 500 and 2000 rotations per minute. In some arrangements, the balloon catheter can be rotated at a speed of between 600 and 1500 rotations per minute. In some arrangements, the balloon catheter can be rotated at a speed of between 700 and 1000 rotations per minute. In some arrangements, the balloon catheter can be rotated at a speed greater than 500, 600, 700, 800, 900, or 1000 rotations per minute.

It will be appreciated that in many arrangements the medical device to be coated is relatively flexible such that if one end is rotated and the other is not the device will twist. Excessive twisting can be detrimental to the quality of the coating disposed thereon. As such, in some arrangements the rotation mechanisms are configured to reduce or eliminate twisting of the medical device. In some arrangements, the rotation mechanisms can include electric motors. In some arrangements, the motors are in electrical communication in order to ensure that they are turning at the same speed, which can help to prevent twisting of the medical device being coated. In some arrangements, only a single electric motor is used and the motive force therefrom is transmitted to both rotation mechanisms. For example, motive force from an electric motor can be transmitted to the rotation mechanisms through components such as gears, drive shafts, belts, and the like.

In some arrangements one or both of the rotation mechanisms can include clutch mechanisms that can be selectively activated. For example, the clutch mechanism can be used to selectively engage or disengage the source of rotational power in one or both of the rotation mechanisms from the medical device to be rotated. An arrangement can be used to begin turning electric motors at a synchronized speed before engaging the clutch to begin rotating the medical device.

In some arrangements, a guide wire, passing through the central lumen of the catheter, can extend from the distal tip of the catheter and be inserted into a distal tip support ring or guide. In this manner, the guide wire can be used to support the distal tip of the balloon catheter to be coated while allowing the balloon catheter to rotate freely. In other arrangements, a connection between the exterior of the catheter and a rotation mechanism can be made directly.

The coating apparatus 600 can further include, in some arrangements, an axial motion mechanism which can be configured to move the balloon catheter in the direction of its lengthwise major axis. In some arrangements, axial motion can be substantially horizontal. In other arrangements, axial motion can be substantially vertical. In some arrangements, axial motion can be somewhere in between horizontal and vertical, depending on the orientation of the lengthwise axis of the balloon catheter. In some arrangements, the axial motion mechanism can be a linear actuator. In some arrangements, the axial motion mechanism can include an electric motor.

The coating apparatus 600 further includes a controller 624 that can serve to control operation of the coating apparatus 600 including, specifically, coating application unit 608, fluid pump 612, rotation mechanism 616, rotation mechanism 618, and/or other components of the system. The controller 624 can include various components such as a processor, memory (such as RAM), input and output channels, power supply, and the like. In some arrangements, the controller 624 can specifically include a motor controller.

It will be appreciated that in various arrangements the coating application unit can move, in other arrangements the rotation mechanisms along with the medical device being coated can move, and in still other arrangements other both the coating application unit and the other components such as the rotation mechanisms can all move with respect to one another.

In various arrangements the coating solution being applied can include a component that is activated such as by actinic radiation. In such arrangements, the coating apparatus can further include an actinic radiation source, such as a UV light source.

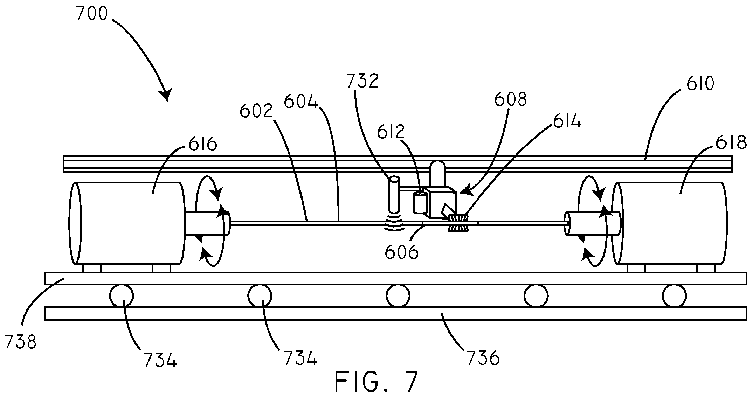

Referring now to FIG. 7, a schematic side view is shown of a coating apparatus 700. In this arrangement, the coating apparatus 700 includes an actinic radiation source 732. The actinic radiation source 732 can include a UV light source. The coating apparatus 700 can further include a mechanism(s) to allow the rotation mechanisms 616, 618 to move in the direction of the lengthwise axis of the medical device. By way of example, the coating apparatus 700 can include rollers 734 that allow the rotation mechanisms and the platform 738 to which they are mounted to move with respect to an underlying support 736.

Fluid Applicator

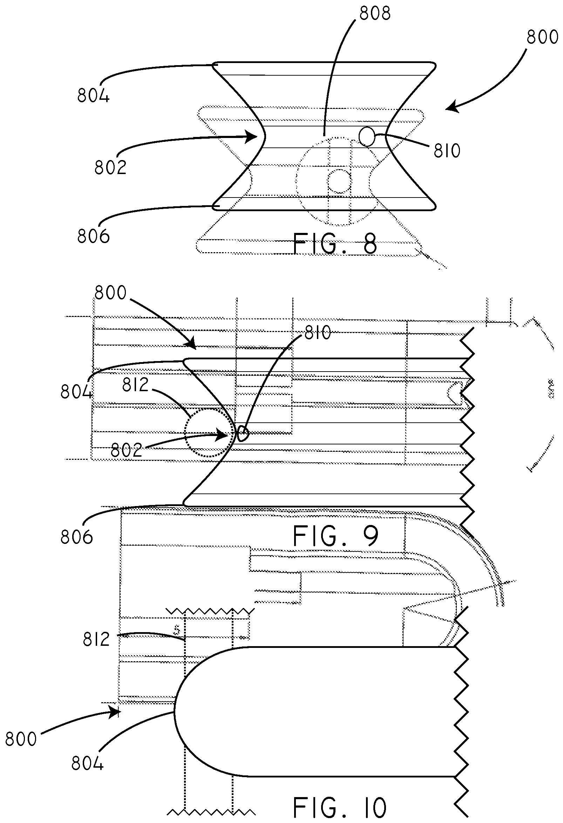

It will be appreciated that the fluid applicator can take on various forms. According to the invention, the fluid applicator is configured to maintain contact with the medical device being coated despite the high rate of rotation. Referring now to FIG. 8, a front elevational view of a fluid applicator 800, which is not part of the invention, is shown. The fluid applicator 800 includes a fluid orifice 810. The fluid orifice 810 can be positioned in various areas. According to the invention, the fluid orifice 810 is positioned at a point away from a lateral center point 808 of the fluid applicator 800. The fluid applicator 800 can include a top 804, a bottom 806, and a middle 802 that is undercut with respect to the top 804 and the bottom 806. As such, the top 804, bottom 806, and middle 802 can form a U-channel. The medical device to be coated can fit within (in whole or in part) with the U-channel. The U-channel can wrap around the entire front of the fluid applicator 800 in some arrangements. Referring now to FIG. 9, a side elevational view of the fluid applicator 800 is shown. In this view, it can be seen that the U-channel can wrap around onto the side of the fluid applicator 800. According to the invention, the U-channel extends along a radius of curvature away from the lateral center point 808. In this view, the perimeter of an exemplary medical device 812 is superimposed into the figure in order to illustrate how the medical device 812 that interface with the fluid applicator 800. Referring now to FIG. 10, a top plan view of the fluid applicator 800 is shown. While not intending to be bound by theory, applicants have found that the type of fluid applicator shown FIGS. 8-10 can be particularly useful in the context of coating processes wherein multiple passes of the coating application unit with respect to the medical device are made.

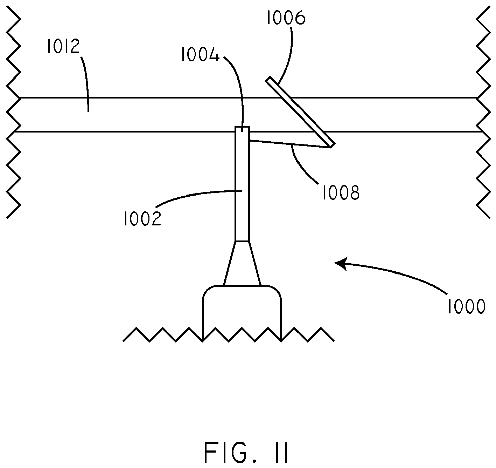

Referring now to FIG. 11, a schematic view of a fluid applicator 1000 is shown, which is not part of the claimed invention. The fluid applicator 1000 includes a fluid conduit 1002 at the end of which is a fluid orifice 1004. The fluid applicator 1000 also can include a connector 1008 and a contact bar 1006. The contact bar 1006 is oriented such that it contacts the medical device to be coated 1012 when the fluid applicator is oriented in a position to apply the coating solution to the medical device 1012. The connector can exhibit a spring force upon movement of the contact bar 1006 relative to the fluid conduit 1002. While the connector 1008 is shown attached to the fluid conduit 1002 in this arrangement, it will be appreciated that the connector 1008 can also be attached to other components of the fluid applicator 1000 and/or the coating apparatus.

Medical Devices

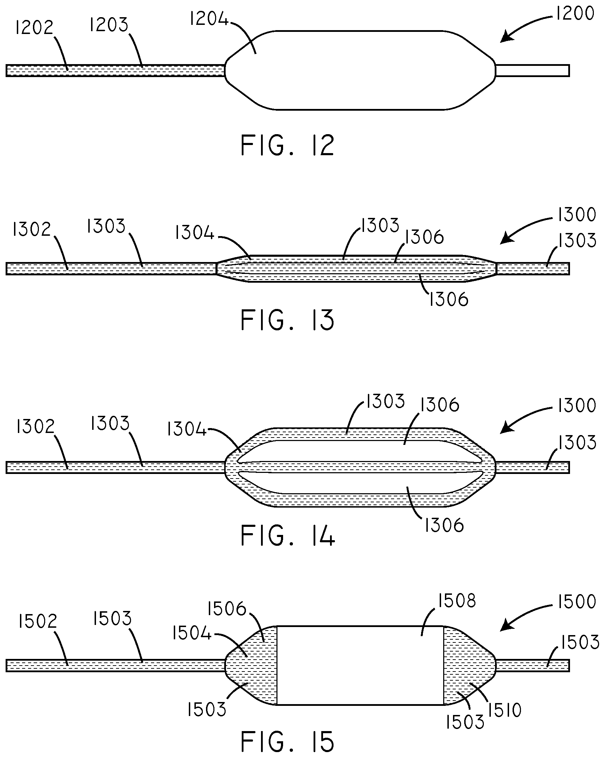

The coating apparatus herein allows the precise application of coating materials onto medical devices with an extraordinary degree of control regarding where the coating stops and starts along the length of the medical device and the amount of coating applied. In addition, the high rotations per minute at which arrangements herein operate allows for certain types of coatings to be applied that are otherwise impossible. Referring now to FIG. 12, a medical device 1200 is shown (in an inflated configuration). The medical device 1200 includes a shaft 1202 and a balloon 1204. A coating material 1203 is disposed on the shaft 1202 on one side of the balloon 1204, but not on the balloon 1204 itself and not on the other side of the balloon.

Referring now to FIG. 13, a medical device 1300 is shown (in a deflated configuration). The medical device 1300 includes a shaft 1302 and a balloon 1304. A coating material is 1303 is disposed on the shaft 1302 and on the balloon 1304. The balloon 1304 includes longitudinal pleats or folds 1306 when it is in a deflated configuration. FIG. 14 shows the same medical device 1300 in an inflated configuration. In this view it can be seen that the coating covers most of the balloon 1304 but does not cover the balloon 1304 in the areas of the longitudinal pleats or folds 1306. As such, the coating layer defines apertures in the areas of the longitudinal pleats.

In some arrangements, only a small amount of coating material is drawn into the area of the pleats. By way of example, the amount of coating material in the area of the pleats (as measured when the balloon is expanded) is less than 10 % for a given amount of surface area than in the fully coated portion of the balloon (such as in the area outside of the longitudinal pleats). In some arrangements, the amount of coating material in the area of the pleats is less than 5 % for a given amount of surface area than in the fully coated portion of the balloon. In some arrangements, the amount of coating material in the area of the pleats is less than 2 % for a given amount of surface area than in the fully coated portion of the balloon. In some arrangements, the amount of coating material in the area of the pleats is less than 1 % for a given amount of surface area than in the fully coated portion of the balloon.

Referring now to FIG. 14, a medical device 1500 is shown (in an inflated configuration). The medical device 1500 includes a shaft 1502 and a balloon 1504. A coating material 1503 is disposed on the shaft 1502 on one side of the balloon 1504, in a first area 1506 on one end of the balloon 1504, in a second area 1510 on the other end of the balloon 1504, and on the shaft 1502 on the other side of the balloon 1504. The coating material 1503 is not disposed in a middle segment 1508 of the balloon.

While examples have been provided in the context of balloon catheters, it will be appreciated that many different types of medical devices can be coated in accordance with arrangements herein. By way of example, medical devices can include, but are not limited to catheters, guide wires, leads, stents, grafts, conduits, or any other type of rotatable medical device or medical device component. In various arrangements, medical devices herein include devices with tubular portions. In various arrangements, medical device herein include rotatable medical devices.

Other medical devices that can be coated using arrangements described herein include medical devices with embolic protection filters on the distal end of the catheter. It can be desirable to have the embolic protection filter either coated with a different coating than the catheter guide wire (e.g. heparin), or the embolic protection filter can remain free of coating. This allows for the device to be economically coated for specific purposes, whereas dip coating, for example, could not accomplish this purpose without tedious masking of the parts that are to remain uncoated.

Yet other medical devices that benefit from the coating methods and apparatus described herein are medical devices that have electrodes or sensors as an integral aspect of the medical device (e.g. glucose sensors, pacemaker leads, etc.). Coatings can impede or change the sensitivity of the sensors or electrodes to yield spurious, inaccurate measurements. Coatings conducted in accordance with the arrangements described herein can produce discontinuous coatings at the site of the sensor or electrode on the medical device, thus avoiding the problem of having the coating interfere with the output measurement of the electrode or sensor.

Some arrangements of the coatings applying the present disclosure can be used to apply discontinuous coatings to a catheter guide wire. For example, the distal tip of a guide wire may beneficially be left uncoated so that the physician can maintain a "feel" when placing the catheter into a human. This "feel" can be changed or completely eliminated if the distal tip of the catheter is coated, for example, with a lubricious material.

Other medical devices that can benefit from coatings described herein include medical devices with small apertures or holes (e.g. atherectomy devices for removal of atherosclerotic tissue such as SILVER-HAWK™ plaque excision system, available from Foxhollow Technologies). In order for these types of medical devices to function properly the small apertures or holes must remain open. In some instances, the use of dip coating on these devices can plug small apertures, thus decreasing the efficiency or rendering useless the function of the medical device. Discontinuous coatings achieved using the arrangements described herein can minimize or eliminate aperture plugging with coating materials.

## Methods

Arrangements herein include methods of applying coatings onto medical devices. In an arrangement, the method can include rotating a medical device with a rotation mechanism. In some arrangements the medical device can be a balloon catheter a shaft and a balloon. In some arrangements, the medical device can be rotated at a speed of between 500 and 2000 rotations per minute. In some arrangements, the medical device can be rotated at a speed of between 600 and 1500 rotations per minute. In some arrangements, the medical device can be rotated at a speed of between 700 and 1000 rotations per minute. According to the invention, the medical device is rotated at a speed greater than 500, according to other embodiments greater than 600, 700, 800, 900, or 1000 rotations per minute.

According to the invention, the rotation mechanism includes a first rotation mechanism and a second rotation mechanism. According to the invention, the methods includes securing the medical device with the first rotation mechanism and the second rotation mechanism.

In some arrangements, the method can include moving the fluid applicator relative to the lengthwise axis of the medical device. In some arrangements, the method can include moving the medical device along its lengthwise axis relative to the fluid applicator. In still other arrangements, both the fluid applicator and the medical device can be moved.

According to the invention, applying a coating solution onto the surface of the balloon with a fluid applicator is accomplished through direct contact between the surface of the device with the fluid applicator. In other arrangements, which are not part of the claimed invention, there is no direct contact between the surface of the device and the fluid applicator.

Based on structural characteristics, certain types of medical device are more difficult to coat than others. By way of example, some devices cannot be easily spin-coated even though they include a long shaft based on their characteristics and shape. For example, devices that have some curvature and cannot be straightened out generally cannot be coated with apparatus that require spinning of the device. As such, these devices have been traditionally coated using techniques such as dip coating. However, dip coating suffers from at least three drawbacks. First, dip coating is a relatively slow process making it expensive. Second, because dip coating requires a large container or vat of material to dip into, there is frequently a large amount of coating material that is wasted in the form of a residual volume in the container into which the device is dipped. Third, dip coating can result in various coating irregularities including thickness variation, webbing, and the like.

Apparatus disclosed herein, but which are not part of the claimed invention, can be used to coat device that would otherwise be coated using dip-coating or device spin-coating techniques. In specific, coating apparatus herein can include a rotating contact member that rotates around the outer diameter of a device to be coated and applying a coating material while the device to be coated remains substantially rotationally stationary. The apparatus can be moved along the lengthwise axis of the device to be coated (and/or the device to be coated can be moved relative to the apparatus) while the rotating contact member rotates around the device to be coated applying the coating. The apparatus can coat the device regardless of shapes such as curvature since only a relatively small length of the device to be coated is in the apparatus at any given time and thus the device does not need to be substantially straight over its entire length as would normally be required if the device were being coated with an apparatus where the device itself was spun.

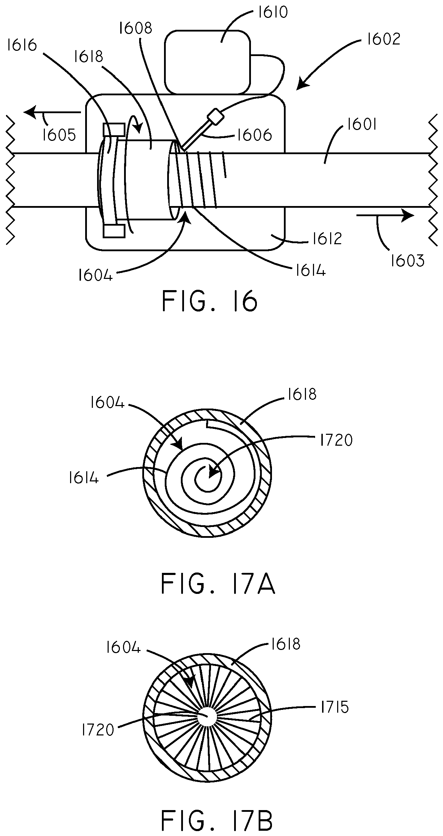

FIG. 16 is a schematic view of a coating apparatus 1602, which is not part of the claimed invention. The coating apparatus 1602 includes a rotating contact member 1604, a fluid applicator 1606, a fluid pump 1610, and a base member 1612. The fluid applicator 1606 includes an orifice 1608. In operation, the fluid pump 1610 can cause a coating solution to pass through the fluid applicator 1606 and out of the orifice 1608 and onto a medical device 1601 to be coated. While FIG. 16 shows the orifice positioned to deposit coating material near the trailing edge of the drive shaft, it will be appreciated that the orifice can also be positioned in other locations to deposit the coating material. Also, while in some arrangements the orifice is on the top side of the medical device, it will be appreciated that it can also be located on the side or the bottom. The rotating contact member 1604 can include a spiral-shaped element 1614. The coating apparatus 1602 can include a mounting structure 1616. The mounting structure 1616 can allow the rotating contact member 1604 to rotate. The mounting structure 1616 can include bearings, bushings, or the like. The coating apparatus 1602 can further include a drive shaft 1618. In some arrangements, the drive shaft 1618 can be a part of the rotating contact member 1604. The medical device 1601 can move in the direction of arrow 1603 with respect to the coating apparatus 1602. Alternatively, the coating apparatus 1602 can move in the direction of arrow 1605 with respect to the medical device 1601. In some arrangements, both the coating apparatus 1602 and the medical device 1601 can move with respect to each other.

FIG. 17A is a front view of a drive shaft and rotating contact member of a coating apparatus. The rotating contact member 1604 can include spiral-shaped element 1614. The rotating contact member 1604 can define a channel 1720. The channel 1720 can be sized to accommodate a medical device 1601 (not shown) as described in FIG. 16. The coating apparatus 1602 can include drive shaft 1618.

In some arrangements, the rotating contact member can include a plurality of bristles and/or a brush. FIG. 17B is a front view of a drive shaft and rotating contact member of a coating apparatus in accordance with various arrangements herein. The rotating contact member 1604 can include bristles 1715. The bristles 1715 can be oriented circumferentially around the rotating contact member 1604 with an inward bias in some arrangements. In some arrangements the bristles 1715 can be connected to drive shaft 1618 or a similar structure that rotates. The rotating contact member 1604 can define a channel 1720. The channel 1720 can be sized to accommodate a medical device 1601 (not shown) as described in FIG. 16.

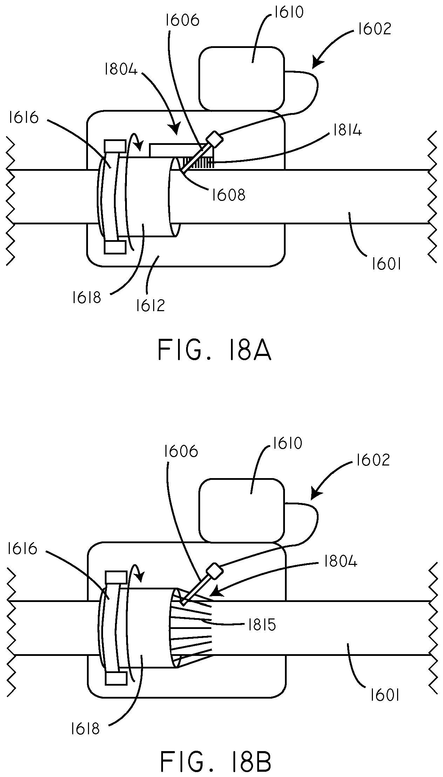

FIG. 18A is a schematic view of a coating apparatus, which is not part of the claimed invention. The coating apparatus 1602 includes a rotating contact member 1804, a fluid applicator 1606, a fluid pump 1610, and a base member 1612. The fluid applicator 1606 includes an orifice 1608. The coating apparatus 1602 can include mounting structure 1616. The coating apparatus 1602 can include drive shaft 1618. The rotating contact member 1804 can include a brush 1814 or similar brush-like structure. The brush 1814 can contact the surface of the medical device 1601 to be coated as the rotating contact member 1804 rotates around and contacts the medical device 1601 to be coated.

FIG. 18B is a schematic view of a coating apparatus, which is not part of the claimed invention. The coating apparatus 1602 includes a rotating contact member 1804, a fluid applicator 1606, a fluid pump 1610, and a base member. The coating apparatus 1602 can include mounting structure 1616. The coating apparatus 1602 can include drive shaft 1618. The rotating contact member 1804 can include bristles 1815 or similar structure. The bristles 1815 can contact the surface of the medical device 1601 to be coated as the rotating contact member 1804 rotates around and contacts the medical device 1601 to be coated.

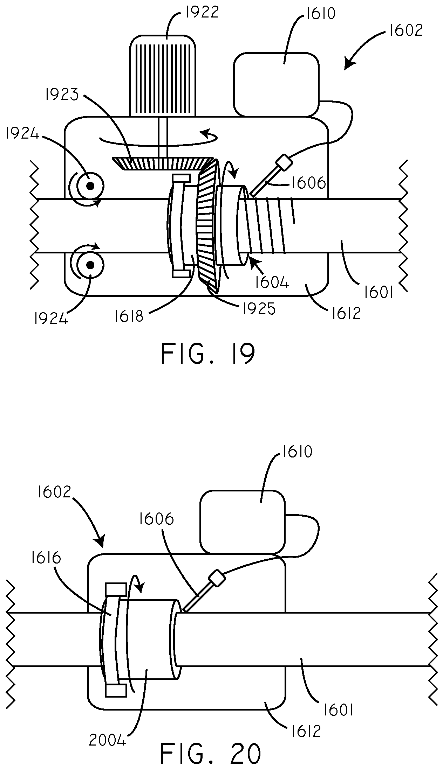

FIG. 19 is a schematic view of a coating apparatus, which is not part of the claimed invention. The coating apparatus 1602 includes a motor 1922, a rotating contact member 1604, a fluid applicator 1606, a fluid pump 1610, and a base member 1612. The fluid applicator 1606 includes an orifice. The coating apparatus 1602 can include a mounting structure. The coating apparatus 1602 can include drive wheels 1924. The drive wheels 1924 can contact the medical device 1601 to be coated and can serve to push or pull the medical device 1601 to be coated through the rotating contact member 1604. The motor 1922 can provide motive force to rotate the rotating contact member 1604 and/or the drive wheels (or shafts) 1924. By way of example, the motor 1922 can be used to turn a drive gear 1923 which can in turn drive an open center gear 1925 causing the rotating contact member 1604 to rotate. However, it will be appreciated that there are many different ways of conveying motive force from the motor 1922 to the rotating contact member 1604 including pulleys, belts, other types of gears, and the like. The motor 1922 can be of many different types. In various arrangements the motor 1922 can be an electric motor. In some arrangements, a motor can be omitted.

FIG. 20 is a schematic view of a coating apparatus, which is not part of the claimed invention. The coating apparatus 1602 includes a rotating contact member 2004, a fluid applicator 1606, a fluid pump 1610, and a base member 1612. The fluid applicator 1606 includes an orifice. The coating apparatus 1602 can include mounting structure 1616. The rotating contact member 2004 can have an inner diameter just slightly larger than the outside of the medical device 1601 to be coated.

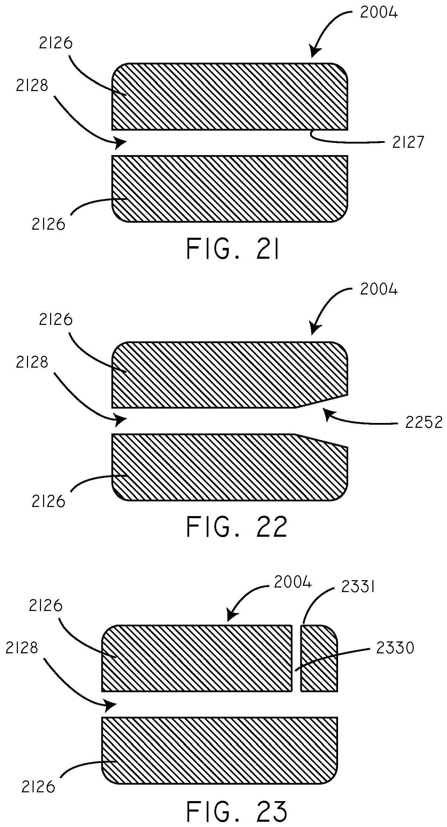

FIG. 21 is a schematic cross-sectional view of a rotating contact member, which is not part of the claimed invention. The rotating contact member 2004 can include a housing 2126. The housing 2126 can define a central lumen 2128. The central lumen 2128 can have a diameter sufficiently large so as to accommodate the outside diameter of the medical device 1601 (not shown) to be coated. In some arrangements, the inner surface 2127 of the central lumen 2128 can be substantially smooth. In other arrangements, the inner surface of the central lumen 2128 can include surface features. In some arrangements, the inner surface of the central lumen 2128 can include thread-like projections similar to the inner diameter of a nut.

In some arrangements, the central lumen can be substantially the same over the length of the rotating contact member. In other arrangements, different portions of the central lumen can be different. Referring now to FIG. 22, the rotating contact member 2004 can include housing 2126. The housing 2126 can define central lumen 2128. In this arrangement, the diameter of central lumen 2128 is larger on one side of the rotating contact member 2004 than on the other. In some arrangements, the central lumen 2128 can have a tapered 2252 or funnel-like shape on one side.

In some arrangements, the coating material can be applied through the fluid applicator. However, in other arrangements, the coating material can be applied through other structures. Referring now to FIG. 23, the rotating contact member 2004 can include a housing 2126. The housing can define central lumen 2128. The housing 2126 can include fluid port 2330. The fluid port 2330 can provide fluid communication between the central lumen 2128 and the exterior surface 2331 of the rotating contact member 2004. A coating composition can be supplied to exterior portion of the fluid port 2330 and can then flow to the central lumen 2128 where it can be applied to a device to be coated. In some arrangements multiple fluid ports 2330 can be provided on the rotating contact member 2004.

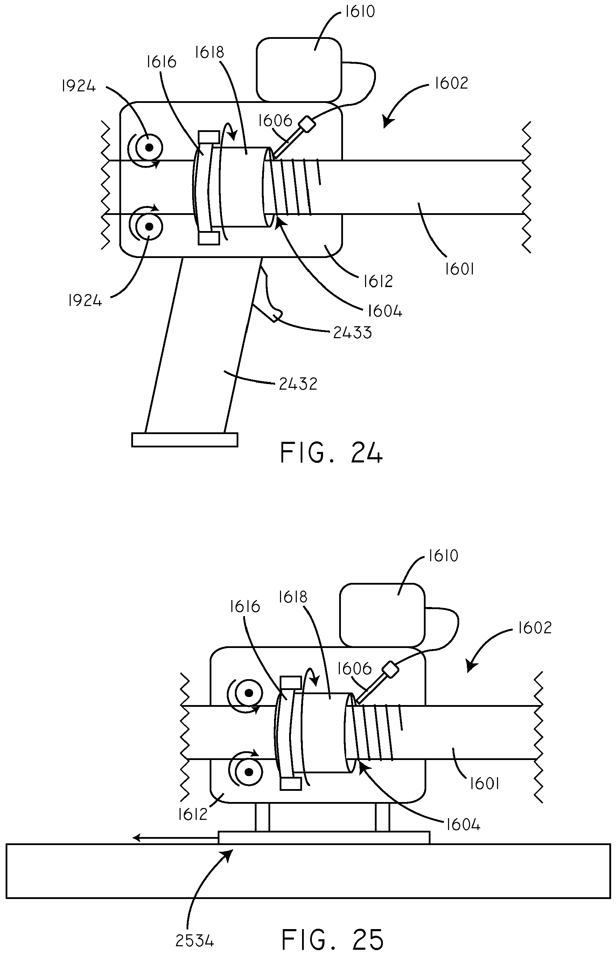

Coating apparatus as described herein can take on various configurations. In some arrangements, which are not part of the claimed invention, the coating apparatus can be hand held. Referring now to FIG. 24, the coating apparatus 1602 includes a rotating contact member 1604, a fluid applicator 1606, a fluid pump 1610, and a base member 1612. The fluid applicator 1606 includes an orifice. The coating apparatus 1602 can include mounting structure 1616. The coating apparatus 1602 can include drive wheels 1924, 1924. The coating apparatus 1602 can include drive shaft 1618. The coating apparatus 1602 can include a hand grip 2432. The hand grip 2432 can include a control element such as a trigger 2433 to control operation of the apparatus.

In some arrangements, which are not part of the claimed invention, the apparatus can be mounted on a structure and move along the lengthwise axis of a device to be coated. Referring now to FIG. 25, the coating apparatus 1602 includes a rotating contact member 1604, a fluid applicator 1606, a fluid pump 1610, and a base member 1612. The fluid applicator 1606 can include an orifice. The coating apparatus 1602 can include a mounting structure 1616. The coating apparatus 1602 comprising drive wheels. The coating apparatus 1602 can include drive shaft 1618. The coating apparatus 1602 can also include a linear actuator 2534. The linear actuator can provide motive force in order to move the coating apparatus linearly so as to provide movement along the lengthwise axis of the device 1601 to be coated.

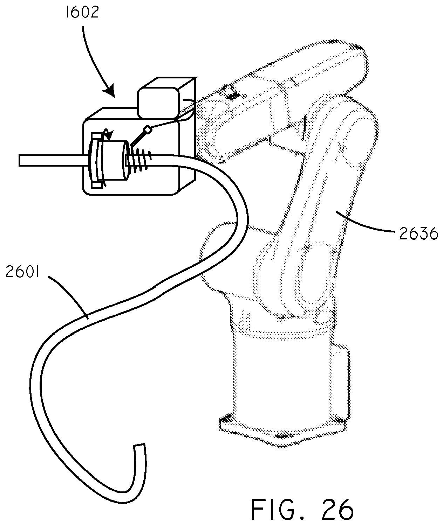

FIG. 26 is a schematic view of a coating apparatus, which is not part of the claimed invention. In this arrangement the coating apparatus 1602 includes a six-axis robot arm 2636. The robot arm 2636 can be used to move the coating apparatus 1602 in such a way that it follows the contours of a medical device 2601 to be coated.

It will be appreciated that the rotating contact member can take on many different shapes and configurations. In some arrangements, the rotating contact member can have a spiral shape. For example, the rotating contact member can be a spiral-shaped element. The spiral-shaped element can include a flexible material. The spiral-shaped element can be formed of various materials including polymers, metals, and the like. In some arrangements, the spiral-shaped element is formed of a shape-memory metal. The spiral of the spiral-shaped element can include at least about two turns. In some arrangements, the spiral-shaped element is arranged so that rotation carries a coating composition along the surface of the device to be coated in the same direction along the lengthwise axis of the device as the rotating contact member moves. In other words, the spiral-shaped element can be used to push the coating material outward ahead of the oncoming rotating contact member versus pull the coating material inward toward the rotating contact member. However, in other arrangements, the orientation of the spiral-shaped element can be reversed so that it pulls the coating material in towards the rotating contact member.

The rotating contact member can include a housing in some arrangements. The housing can be made of many different materials including metals, polymers, composites, ceramics, and the like. In some arrangements, the housing can be formed of polytetrafluoroethylene. The housing can define a central lumen into which the device to be coated fits. The central lumen can have a larger diameter at one end than at the other. The central lumen can form a funnel shape in some arrangements. The funnel shape can be disposed at one end of the housing. The housing can also define a fluid port in some arrangements. The housing can be cylindrical in some arrangements

The rotating contact member can rotate at a speed in the range of about 50 to 400 RPM. The rotating contact member can rotate at a speed of about 100 to 200 RPM. In some arrangements, the rotating contact member can rotate at a speed of greater than about 50 RPM. In some arrangements, the rotating contact member can rotate at a speed of greater than about 75 RPM. In some arrangements, the rotating contact member can rotate at a speed of greater than about 100 RPM. In some arrangements, the rotating contact member can rotate at a speed of greater than about 125 RPM. In some arrangements, the rotating contact member can rotate at a speed of less than about 400 RPM. In some arrangements, the rotating contact member can rotate at a speed of less than about 350 RPM. In some arrangements, the rotating contact member can rotate at a speed of less than about 275 RPM. In some arrangements, the rotating contact member can rotate at a speed of less than about 200 RPM.

The rotating contact member and/or the channel can be sized to accommodate a device to be coated having a diameter of between 0.5 mm and 20 mm. In some arrangements, the rotating contact member and/or the channel can accommodate a device to be coated having a diameter greater than about 0.5 mm. In some arrangements, the rotating contact member and/or the channel can accommodate a device to be coated having a diameter greater than about 1 mm. In some arrangements, the rotating contact member and/or the channel can accommodate a device to be coated having a diameter greater than about 3 mm. In some arrangements, the rotating contact member and/or the channel can accommodate a device to be coated having a diameter less than about 15 mm. In some arrangements, the rotating contact member and/or the channel can accommodate a device to be coated having a diameter less than about 11 mm. In some arrangements, the rotating contact member and/or the channel can accommodate a device to be coated having a diameter less than about 8 mm. In some arrangements, the rotating contact member and/or the channel can accommodate a device to be coated having a diameter between about 0 mm and about 15 mm. In some arrangements, the rotating contact member and/or the channel can accommodate a device to be coated having a diameter between about 1 mm and about 11 mm. In some arrangements, the rotating contact member and/or the channel can accommodate a device to be coated having a diameter between about 3 mm and about 8 mm.

The coating apparatus can include drive wheels in various arrangements (such as that shown in FIG. 24. The drive wheel can contact the device to be coated can generate force to push or pull the device to be coated through the apparatus. In some arrangements, the drive wheel pulls the device to be coated through the rotating contact member. In some arrangements, the drive wheel can be substantially smooth. In some arrangements, the drive wheel can include a surface texture. The drive wheel can be formed of various materials. In some arrangements, the drive wheel can be formed of silicone (PMDS).

The apparatus can move along the lengthwise axis of the device to be coated at various speeds through the action of the drive wheels or another source of motive force. In some arrangements, the apparatus can coat that device to be coated at a speed of between 0.1 and 1.5 cm per second.

In some arrangements, the apparatus can coat a device to be coated at a speed of greater than 0.1 cm/s. In some arrangements, the apparatus can coat a device to be coated at a speed of greater than about 0.5 cm/s. In some arrangements, the apparatus can coat a device to be coated at a speed of greater than about 1.0 cm/s. In some arrangements, the apparatus can coat a device to be coated at a speed of less than about 2 cm/s. In some arrangements, the apparatus can coat a device to be coated at a speed of less than about 1.5 cm/s. In some arrangements, the apparatus can coat a device to be coated at a speed of less than about 1 cm/s. In some arrangements, the apparatus can coat a device to be coated at a speed of between about 0 cm/s and about 2 cm/s. In some arrangements, the apparatus can coat that device to be coated at a speed of between about 0.1 cm/s and about 1.5 cm/s. In some arrangements, the apparatus can coat that device to be coated at a speed of between about 0.5 cm/s and about 1 cm/s.

In some arrangements, the rotating contact member can assume an open configuration and a closed configuration. In some arrangements, the device to be coated can be inserted or removed from the rotating contact member when it is in the open configuration.

In some arrangements, the coating apparatus can include a drive shaft. The drive shaft conveys motive force between the motor and the rotating contact member. The drive shaft can be hollow. In some arrangements, the device to be coated can be disposed within the rotating contact member and/or the drive shaft such that the rotating contact member and/or the drive shaft rotates around the device to be coated.

Described herein is a method of coating a medical device. This method is not part of the claimed invention. The method of coating a medical device can include rotating a contact member around the outer diameter of a non-rotating medical device. The method can further include applying a coating solution to the outer diameter of the non-rotating medical device at a position adjacent to the contact member. The method can further include moving at least one of the contact member and the non-rotating medical device with respect to one another so that the contact member moves with respect to the lengthwise axis of the non-rotating medical device.

In some arrangements, rotation of the spiral shaped contact member causes the coating composition to move along the surface of the non-rotating medical device in the same direction along the lengthwise axis of the non-rotating medical device as the rotating contact member moves. In some arrangements, applying a coating solution comprises applying the coating solution onto the rotating contact member. In other arrangements, applying a coating solution comprise applying the coating solution directly onto the device to be coated. In some arrangements, an operation of inserting the non-rotating medical device into the contact member can be performed before the step of rotating the contact member.

## Medical Devices

The coating apparatus of arrangements herein allows the precise application of coating materials onto medical devices with an extraordinary degree of control regarding where the coating stops and starts along the length of the medical device, uniformity of the coating applied and the amount of coating applied.

Many different types of medical device can be coated with apparatus described herein. By way of example, medical devices coated in accordance with arrangements described herein can include devices having a degree of curvature and/or stiffness such that they cannot practically be spun- or dip-coated. In a particular arrangement, the device can be one including a curved shaft. In some arrangements, the device can be one that lacks a central lumen.

In some arrangements, the present apparatus and coating methods can be used to coat catheters for transaortic valve implants (TAVI; see SAPEIN trancatheter heart valve; available from Edwards Lifesciences Corporation, Irvine, California). TAVI devices and procedures can be used in cases where patients have severe aortic stenosis but where those patients are not candidates for surgery. TAVI catheters typically are not straight and have three dimensional bends or curves. The catheters are curved in order to assist the physician in the accurate placement of the valve at the site of the stenosis. There is a need to apply hydrophilic coatings to TAVI catheters to improve lubricity upon delivery of the TAVI to the site.

In the past, coating these non-linear, highly curved catheters using traditional methods such as dip or spray coating has resulted in coatings that are inconsistently applied or that require inordinate amount of waste coating material compared to the coating material applied to the surface of the medical device. Apparatus and methods of the present disclosure can be used to accurately apply a coating to the surface of TAVI devices with bends and curves, since apparatus and methods disclosed herein are largely not dependent upon the spatial configuration of the medical device to achieve accurate surface coatings.

In yet other arrangements, the medical device to be coated can be a balloon catheter. The balloon catheter can be coated in the apparatus described herein in the collapsed state. Alternatively, the balloon catheter can be coated in the apparatus described herein in the partially or fully expanded state. In one arrangement, the balloon catheter can be coated with a bioactive material such as a chemical ablative (e.g. vincristine, paclitaxel) and used for renal artery denervation therapy for hypertension.

## Coating Solutions

It will be appreciated that coating solutions used in conjunction with arrangements herein can include various components including, but not limited to, one or more active agents, carrier agents, solvents (aqueous and/or non-aqueous), polymers (including degradable or non-degradable polymers), monomers, macromers, excipients, photoreactive compounds, linking agents, and the like. The relative amounts of the components of the coating solution will depend on various factors.

The coating solutions can be formulated so as to provide various functional properties to the medical device to which they are applied. By way of example, the coating solutions can be formulated so as to provide lubricious properties; anti-infective properties, therapeutic properties, durability and the like.

Viscosities of coating solutions used in conjunction with arrangements herein can vary. In some arrangements, the coating solution is relatively viscous. By way of example, in some arrangements, the coating solution can have a viscosity of 50, 100, 300, 500, 1000, 5000, or 10,000 centipoise or greater. In some arrangements, the coating solution can have a viscosity of between about 50 and 5000 centipoise.

In other arrangements, the coating solution has relatively low viscosity. By way of example, in some arrangements, the coating solution can have viscosity of less than about, 100, 50, 40, 30, 20, or 10 centipoise. In some arrangements, the coating solution can have a viscosity of between about 1 and 100 centipoise, or 1 and 50 centipoise.

In some arrangements, the coating solution can have a solids content that is relatively low. By way of example, in some arrangements, coating solutions used in conjunction with arrangements herein can have a solids content of less than about 10 mg/ml. In some arrangements, coating solutions used in conjunction with arrangements herein can have a solids content of less than about 5 mg/ml. In some arrangements, coating solutions used in conjunction with arrangements herein can have a solids content of less than about 2 mg/ml.

It should be noted that, as used in this specification and the appended claims, the singular forms "a," "an," and "the" include plural referents unless the content clearly dictates otherwise. Thus, for example, reference to a composition containing "a compound" includes a mixture of two or more compounds. It should also be noted that the term "or" is generally employed in its sense including "and/or" unless the content clearly dictates otherwise.

It should also be noted that, as used in this specification and the appended claims, the phrase "configured" describes a system, apparatus, or other structure that is constructed or configured to perform a particular task or adopt a particular configuration to. The phrase "configured" can be used interchangeably with other similar phrases such as arranged and configured, constructed and arranged, constructed, manufactured and arranged, and the like.

The invention has been described with reference to various specific and preferred embodiments and techniques. However, it should be understood that many variations and modifications may be made while remaining within the scope of the invention as defined in the appended claims.

**Claims**

1. A coating apparatus (600, 700) comprising:

    a coating application unit (608) comprising:

        a fluid applicator (800);
        a first rotation mechanism (616) and a second rotation mechanism (618); and
        a controller (624), wherein the controller causes the first rotation mechanism (616) and the second rotation mechanism (618) to rotate a medical device at substantially the same speed, wherein the speed is greater than 500 rotations per minute;

    **characterised in that**,
    the fluid applicator (800) comprises a contact fluid applicator; and **in that**,
    the fluid applicator (800) comprises a U-channel having a radius of curvature away from a lateral center point (808), the fluid applicator comprising a fluid orifice (810), wherein the fluid orifice (810) is disposed on the fluid applicator (800) at a point away from the lateral center point (808) of the fluid applicator (800).

2. The coating apparatus of any of claims 1 and 3-11, wherein the coating application unit (608) is stationary and the first rotation mechanism (616) and second rotation mechanism (618) move in the direction of the lengthwise axis of the medical device.

3. The coating apparatus of any of claims 1-2 and 4-11, wherein first rotation mechanism (616) and second rotation mechanism (618) are stationary and the coating application unit (608) moves in the direction of the lengthwise axis of the medical device.

4. The coating apparatus of any of claims 1-3 and 5-11, further comprising an electric motor providing power to at least one of the first and second rotation mechanisms (616, 618).

5. The coating apparatus of any of claims 1-4 and 6-11, further comprising a fluid pump (612) in fluid communication with the fluid applicator (800).

6. The coating apparatus of any of claims 1-5, wherein the first rotation mechanism (616) and second rotation mechanism (618) are configured to rotate a balloon catheter.

7. A method of coating with the apparatus of claim 1, the method comprising:

    rotating a medical device with the first and the second rotation mechanism (616, 618) at a speed of greater than 500 rotations per minute; contacting the medical device with the fluid applicator (800), the fluid applicator (800) comprising a contact fluid applicator; and applying a coating solution to the device with the fluid applicator (800), the method further comprising securing the medical device with the first rotation mechanism (616) and the second rotation mechanism (618).

8. The method of any of claims 7 and 9-11, further comprising moving the fluid applicator (800) along the lengthwise axis of the medical device.

9. The method of any of claims 7-8 and 10-11, wherein the medical device comprises a balloon and wherein applying a coating solution onto the surface of the balloon with a fluid applicator (800) is accomplished through direct contact between the surface of the device with the fluid applicator (800).

10. The method of any of claims 7-9 and 11, wherein the coating solution has a viscosity of between 1 and 50 centipoise.

11. The method of any of claims 7-10, wherein the coating solution has a viscosity of between 50 and 5000 centipoise.

## Patentansprüche

1. Beschichtungsvorrichtung (600, 700), umfassend:

   eine Beschichtungsauftragseinheit (608), umfassend:

   einen Fluidapplikator (800);
   einen ersten Rotationsmechanismus (616) und einen zweiten Rotationsmechanismus (618); und
   eine Steuerung (624), wobei die Steuerung bewirkt, dass der erste Rotationsmechanismus (616) und der zweite Rotationsmechanismus (618) ein Medizingerät mit im Wesentlichen derselben Geschwindigkeit drehen, wobei die Geschwindigkeit höher als 500 Umdrehungen pro Minute ist;

   **dadurch gekennzeichnet, dass** der Fluidapplikator (800) einen Kontaktfluidapplikator umfasst; und
   dass der Fluidapplikator (800) einen U-Kanal mit einem Krümmungsradius entfernt von einem seitlichen Mittelpunkt (808) umfasst, wobei der Fluidapplikator eine Fluidöffnung (810) umfasst, wobei die Fluidöffnung (810) auf dem Fluidapplikator (800) an einer Stelle angeordnet ist, die vom seitlichen Mittelpunkt (808) des Fluidapplikators (800) entfernt ist.

2. Beschichtungsvorrichtung nach einem der Ansprüche 1 und 3 bis 11, wobei die Beschichtungsauftragseinheit (608) stationär ist und der erste Rotationsmechanismus (616) und der zweite Rotationsmechanismus (618) sich in die Richtung der Längsachse des Medizingeräts bewegen.

3. Beschichtungsvorrichtung nach einem der Ansprüche 1 bis 2 und 4 bis 11, wobei der erste Rotationsmechanismus (616) und der zweite Rotationsmechanismus (618) stationär sind, und sich die Beschichtungsauftragseinheit (608) in die Richtung der Längsachse des Medizingeräts bewegt.

4. Beschichtungsvorrichtung nach einem der Ansprüche 1 bis 3 und 5 bis 11, ferner umfassend einen Elektromotor, der zumindest einen des ersten und des zweiten Rotationsmechanismus (616, 618) mit Leistung versorgt.

5. Beschichtungsvorrichtung nach einem der Ansprüche 1 bis 4 und 6 bis 11, ferner umfassend eine Fluidpumpe (612) in Fluidverbindung mit dem Fluidapplikator (800).

6. Beschichtungsvorrichtung nach einem der Ansprüche 1 bis 5, wobei der erste Rotationsmechanismus (616) und der zweite Rotationsmechanismus (618) dafür konfiguriert sind, einen Ballonkatheter zu drehen.

7. Verfahren zum Beschichten mit der Vorrichtung nach Anspruch 1, das Verfahren umfassend:

   Drehen eines Medizingeräts mit dem ersten und zweiten Rotationsmechanismus (616, 618) mit einer Geschwindigkeit von mehr als 500 Umdrehungen pro Minute;
   Inkontaktbringen des Medizingeräts mit dem Fluidapplikator (800), wobei der Fluidapplikator (800) einen Kontaktfluidapplikator umfasst; und
   Auftragen einer Beschichtungslösung auf das Produkt mit dem Fluidapplikator (800), wobei das Verfahren ferner das Sichern des Medizingeräts mit dem ersten Rotationsmechanismus (616) und dem zweiten Rotationsmechanismus (618) umfasst.

8. Verfahren nach einem der Ansprüche 7 und 9 bis 11, ferner umfassend das Bewegen des Fluidapplikators (800) entlang der Längsachse des Medizingeräts.

9. Verfahren nach einem der Ansprüche 7 bis 8 und 10 bis 11, wobei das Medizingerät einen Ballon umfasst und wobei das Auftragen einer Beschichtungslösung auf die Oberfläche des Ballons mit einem Fluidapplikator (800) durch direkten Kontakt zwischen der Oberfläche des Geräts und dem Fluidapplikator (800) erreicht wird.

10. Verfahren nach einem der Ansprüche 7 bis 9 und 11, wobei die Beschichtungslösung eine Viskosität zwischen 1 und 50 Centipoise aufweist.

11. Verfahren nach einem der Ansprüche 7 bis 10, wobei die Beschichtungslösung eine Viskosität zwischen 50 und 5000 Centipoise aufweist.

## Revendications

1. Appareil de revêtement (600, 700) comprenant : une unité d'application de revêtement (608) comprenant :

    un applicateur de fluide (800) ;
    un premier mécanisme de rotation (616) et un second mécanisme de rotation (618) ; et
    un dispositif de commande (624), dans lequel le dispositif de commande amène le premier mécanisme de rotation (616) et le second mécanisme de rotation (618) à faire pivoter un dispositif médical sensiblement à la même vitesse, la vitesse étant supérieure à 500 rotations par minute ;

    **caractérisé en ce que**
    l'applicateur de fluide (800) comprend un applicateur de fluide de contact ; et **en ce que** l'applicateur de fluide (800) comprend un canal en U ayant un rayon de courbure éloigné d'un point central latéral (808), l'applicateur de fluide comprenant un orifice de fluide (810), dans lequel l'orifice de fluide (810) est disposé sur l'applicateur de fluide (800) en un point éloigné du point central latéral (808) de l'applicateur de fluide (800).

2. Appareil de revêtement selon l'une quelconque des revendications 1 et 3 à 11, dans lequel l'unité d'application de revêtement (608) est stationnaire et le premier mécanisme de rotation (616) et le second mécanisme de rotation (618) se déplacent dans la direction de l'axe longitudinal du dispositif médical.

3. Appareil de revêtement selon l'une quelconque des revendications 1 à 2 et 4 à 11, dans lequel le premier mécanisme de rotation (616) et le second mécanisme de rotation (618) sont stationnaires et l'unité d'application de revêtement (608) se déplace dans la direction de l'axe longitudinal du dispositif médical.

4. Appareil de revêtement selon l'une quelconque des revendications 1 à 3 et 5 à 11, comprenant en outre un moteur électrique fournissant de l'énergie à au moins l'un des premier et second mécanismes de rotation (616, 618).

5. Appareil de revêtement selon la revendication 1 à 4 et 6 à 11, comprenant en outre une pompe à fluide (612) en communication fluidique avec l'applicateur de fluide (800).

6. Appareil de revêtement selon l'une quelconque des revendications 1 à 5, dans lequel le premier mécanisme de rotation (616) et le second mécanisme de rotation (618) sont conçus pour faire pivoter un cathéter à ballonnet.

7. Procédé de revêtement avec l'appareil selon la revendication 1, le procédé comprenant :

    la rotation d'un dispositif médical doté de premier et second mécanismes de rotation (616, 618) à une vitesse supérieure à 500 rotations par minute ;
    la mise en contact du dispositif médical avec l'applicateur de fluide (800), l'applicateur de fluide (800) comprenant un applicateur de fluide de contact ; et
    l'application d'une solution de revêtement au dispositif avec l'applicateur de fluide (800), le procédé comprenant en outre la fixation du dispositif médical doté du premier mécanisme de rotation (616) et du second mécanisme de rotation (618).

8. Procédé selon l'une des revendications 7 et 9 à 11, comprenant en outre le déplacement de l'applicateur de fluide (800) le long de l'axe longitudinal du dispositif médical.

9. Procédé selon l'une des revendications 7 à 8 et 10 à 11, dans lequel le dispositif médical comprend un ballon et dans lequel l'application d'une solution de revêtement sur la surface du ballon avec un applicateur de fluide (800) est réalisée par contact direct entre la surface du dispositif et l'applicateur de fluide (800).

10. Procédé selon l'une quelconque des revendications 7 à 9 et 11, dans lequel la solution de revêtement a une viscosité comprise entre 1 et 50 centipoises.

11. Procédé selon l'une quelconque des revendications 7 à 10, dans lequel la solution de revêtement a une viscosité comprise entre 50 et 5000 centipoises.

FIG. I

FIG. 2A

FIG. 2B

300

302

306

304

FIG. 3

416

414

400

FIG. 4

FIG. 5A

FIG. 5B

FIG. 6

FIG. 7

FIG. 8

FIG. 9

FIG. 10

FIG. II

1202    1203        1204                                                    1200

FIG. 12

1302    1303        1304                1303    1306        1300
                                                            1303
                                1306

FIG. 13

1302    1303        1304            1303    1306        1300
                                                        1303
                                                1306

FIG. 14

1502    1503    1506            1508        1500
        1504                                1503
1503                        1503    1510

FIG. 15

FIG. 16

FIG. 17A

FIG. 17B

FIG. 18A

FIG. 18B

FIG. 19

FIG. 20

2004

2126

2128

2126

2127

FIG. 21

2004

2126

2128

2126

2252

FIG. 22

2004    2331

2126

2128

2330

2126

FIG. 23

FIG. 24

FIG. 25

FIG. 26

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 4195637 A
- US 5041089 A
- US 5087246 A
- US 5318587 A
- US 5382234 A
- US 5571089 A
- US 5776101 A
- US 5807331 A
- US 5882336 A
- US 6394995 B
- US 6517515 B
- US 6623504 B
- US 6896842 B
- US 7163523 B
- US 20110311713 A
- US 20100070020 A
- US 20100055294 A
- US 20090018643 A
- US 4743252 A
- US 20110238011 A
- US 20050233061 A